(19) Europäisches Patentamt
European Patent Office
Office européen des brevets

(11) Publication number: **0 010 466**
**B1**

(12) **EUROPEAN PATENT SPECIFICATION**

(45) Date of publication of patent specification: **09.02.83**

(21) Application number: **79400681.7**

(22) Date of filing: **25.09.79**

(51) Int. Cl.[3]: **B 01 J 31/06,**
C 07 D 333/38,
C 08 F 8/32, C 08 F 8/40,
C 08 F 8/44

(54) **Polymer bound phase transfer catalyst and process for its preparation.**

(30) Priority: **02.10.78 US 947611**

(43) Date of publication of application:
**30.04.80 Bulletin 80/9**

(45) Publication of the grant of the patent:
**09.02.83 Bulletin 83/6**

(84) Designated Contracting States:
**BE CH DE FR GB**

(56) References cited:
FR - A - 1 477 003
US - A - 3 168 502
US - A - 4 105 642

(73) Proprietor: **ETHYL CORPORATION**
**330 South Fourth Street P.O. Box 2189**
**Richmond Virginia 23219 (US)**

(72) Inventor: **Bauer, Dennis Paul**
**3650 - 2179 Nicholson Drive**
**Baton Rouge, Louisiana 70802 (US)**

(74) Representative: **Rinuy, Guy et al,**
**14, Avenue de la Grande Armée**
**F-75017 Paris (FR)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

**EP 0 010 466 B1**

Courier Press, Leamington Spa, England

Polymer bound phase transfer catalyst and process for its preparation

This invention deals with an improved process for converting 3-thenylbromide to 3-thienylacetonitrile by reaction of the first of these compounds with an alkali metal cyanide and in the presence of triphase reaction catalysts. Further, new catalyst compositions are provided.

A significant and recurring problem in organic synthesis stems from the use, or desired use, of a water-soluble reagent in chemically altering a water-insoluble organic substrate. If the reaction is conducted as a heterogeneous process (e.g., organic phase-aqueous phase reaction) observed reaction rates are normally very slow owing to the low concentration of at least one of the reactants in each phase. Techniques currently available to circumvent this problem rely on the use of rapid stirring, cosolvent, and phase-transfer methods. If chemical reaction takes place at a liquid-liquid phase boundary, rapid stirring may have an accelerating effect by increasing interfacial contact. Alternatively, the addition of a cosolvent can bring about a homogeneous state and thereby completely eliminate phase separation. Although this latter approach is often useful, product mixtures are necessarily made more complex and the resulting workup made more difficult. In addition, with aqueous phase-organic phase reactions, use of a cosolvent not only renders the organic substrate accessible to the reagent, but also increases the substrate's contact with water and can promote competing hydrolytic pathways. Recently, a third technique has been developed which appears to have considerable potential; this method has been referred to as phase-transfer catalysis. In brief, an organic-soluble, partially water-soluble catalyst (most commonly a tetraalkyl-ammonium or tetraalkylphosphonium salt) accelerates an aqueous phase-organic phase reaction, presumably, by extracting a given ionic reagent out of water and into the bulk organic phase where reaction can ensue. Based on enhanced reaction rates, high yields of products, and the convenience found with this method, it seems likely that many industrial applications will be forthcoming. One practical limitation to the phase-transfer method, however, is that many of the catalysts used promote the formation of stable emulsions.

More recently, a new type of heterogeneous catalysis termed "triphase catalysis" has been introduced. The underlying feature which distinguishes this form from other forms of heterogeneous catalysis is that the catalyst and each one of a pair of reactants are located in separate phases. This principle has been successfully applied to certain aqueous phase-organic phase reactions employing a solid phase catalyst. S. L. Regen, *J. Am. Chem. Soc., 97,* 5956 (1975). The present invention relates to a novel catalyst for use in such reactions.

Previously, such triphase catalysts have been used to catalyze aqueous phase-organic phase reactions in which homogeneous phase transfer catalysts have produced stable emulsions, greatly reducing the usefulness of homogeneous phase transfer catalysts. Triphase catalysts have been used in cyanide displacement, halogen exchange, dichlorocarbene addition, dichlorocarbene chlorination, alkoxide and phenoxide displacement, dehalogenation of *vic*-dibromides and oxidation of alcohols. S. L. Regen, *J. Org. Chem. Soc.,* Vol. *42,* No. 5, 1977. In general, the triphase catalyst employ chloromethylated polystrene beads, subsequently reacted with dimethylbutylamine or trimethyl-amine. Dimethyloctylamine, dodecylamine, tetradecylamine, triethylamine, dimethyltoluoylamine and hydroxyethylamine have also been employed to produce triphase catalysts. S. L. Regen, *J. Am. Chem. Soc., 98* 6270 (1976). While such catalysts are known, it is always desirable to find cheaper and more effective materials to produce triphase catalysts . Accordingly, the present invention has as its object the provision of new triphase or polymer bound phase transfer catalysts, and the provision of an improved process to convert 3-thienylbromide to 3-thienylacetonitrile.

The catalysts now provided are represented as

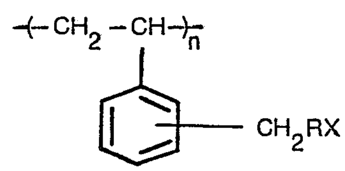

is employed, wherein R, in the above formula, is selected from diethyl-*n*-butylamino, tripropylamino, tributylamino, trialkylphosphino and triarylphosphino; X is chlorine or bromine; and *n* can range from 600 to 2,000,000.

Such catalysts can be prepared by establishing a mixture of chloromethylated styrene polymer in a solvent capable of swelling the polymer, maintaining the mixture until the polymer is swollen, reacting the swollen polymer with an amine or phosphine and recovering the resultant catalyst composition from the reaction mixture.

Such catalyst compositions, as indicated above, have been found to be less expensive and more efficient in the synthesis of nitriles, for example, producing 3-thienylacetonitrile from 3-thenylbromide by reacting in an organic solvent with aqueous alkali metal cyanide.

As indicated, the catalyst composition contains a polymer. It has been found that polystyrene is an

excellent polymer substrate for preparation of triphase or polymer bound phase transfer catalysts. Commercially available polystyrenes can be employed. Reference is made to the article entitled "Styrene Plastics", *Encyclopedia of Chemical Technology* 2nd Ed., *19,* 85—134, Interscience Publishers, New York (1969). Preferably, the polystyrene is crosslinked with from 1—10% of divinylbenzene. Further, the polystyrene is chloromethylated, according to known procedures, so that each of the styrene groups on the polymer contains a chloromethane group. Such chloromethylated polystyrene crosslinked with varying amounts of divinylbenzene is available in bead form from Bio-Rad Laboratories, Richmond, California, designated as "Bio-Beads S—XI", Catalog No. 154—1342.

The chloromethylated polystyrene beads can be reacted with diethyl-n-butylamine, tripropylamine, tributylamine or trialkyl- or triarylphosphines to produce quaternary ammonium and phosphonium groups on the polystyrene resin. As used herein, the term "alkyl" means lower alkyl having from 1 to 4 carbon atoms such as methyl, ethyl, propyl, and butyl; and the term "aryl" means mononuclear unsubstituted and alkyl substituted aromatic groups having from 6 to 10 carbon atoms, defined hereinbefore. In order to effect such a reaction, the polymer is first swelled in a suitable solvent such as aromatric hydrocarbon solvents, for example, benzene, toluene and xylene, or heterocyclic ethers, such as tetrahydrofuran. Preferably, tetrahydrofuran is employed. The swelling can be accomplished by mixing the chloromethylated polystyrene beads with a suitable amount of tetrahydrofuran and allowing the mixture to stand for 10 to 30 hours. The swelled polymer is then separated from the suspension, washed with tetrahydrofuran and air dried for subsequent reaction with an amine or phosphine. This drying step is optional and the swollen polymer beads can be directly reacted with a suitable amine or phosphine after swelling, but on many occasions an amount of swollen beads are prepared for subsequent reaction so that drying is advantageous to maintain the swollen polymer beads in readiness for such subsequent reaction.

The dried, swollen polymer beads can be easily reacted with a suitable amine or phosphine, such as, tri-*n*-butylamine or tri-*n*-butylphosphine, triphenylphosphine or tripropylamine, in a suitable solvent by reacting, under reflux, with the resin under a nitrogen atmosphere for 12—36 hours. After reflux, the reaction mixture is filtered and washed with a mixture of water and tetrahydrofuran and vacuum dried.

The catalyst can be employed to convert bromides to nitriles by reaction in a three-phase system having an organo bromide in an organic solvent and an alkali metal cyanide in an aqueous system. For example, 3-thenylbromide can be converted into 3-thienylacetonitrile by reacting in the presence of a triphase or polymer bound phase transfer catalyst of this invention. Also, 1-bromooctane can be converted into 1-cyanooctane by reaction with a catalyst of this invention. The preparation of triphase catalyst compositions and their use in converting bromides to nitriles is more particularly illustrated in the following examples.

Example 1—Comparative

Into a 200 ml beaker was placed 10 grams of chloromethylated polystyrene beads crosslinked with 1% by weight of divinylbenzene purchased from Bio-Rad Laboratories, Richmond, California and designated as "Bio-Beads S—X1", Catalog No. 154—1342. To the beaker was added 50 ml of dry, distilled tetrahydrofuran and the polymer was "swelled" for 20 hours. The polymer-tetrahydtofuran suspension was then filtered, washed with two fresh, 50 ml portions of tetrahydrofuran and the polymer was air dried for 5 minutes.

The dried, swollen polymer beads were placed into a 300 ml round bottom flask together with 50 ml of tetrahydrofuran and 10 ml of N,N-dimethylbutylamine. The mixture was refluxed under a nitrogen atmosphere for 24 hours. After reflux the reaction mixture was filtered and the solid catalyst was washed with a mixture of 20 ml water and 20 ml tetrahydrofuran and finally with another 20 ml of tetrahydrofuran. The catalyst was vacuum dried.

Example 2

In a procedure similar to Example 1, except that 10 ml of N,N-dimethylbutylamine was replaced by 10 ml of tri-*n*-butylamine, there was produced a polystyrene/tri-*n*-butylammonium chloride catalyst.

Example 3

In a procedure similar to Example 1, except that 10 ml of N,N-dimethylbutylamine was replaced by 10 ml of tri-*n*-butylphosphine, there was produced a polystyrene/tri-*n*-butylphosphonium chloride catalyst.

Example 4

In a procedure similar to Example 1, except that 10 ml of N,N-dimethylbutylamine was replaced by 10 ml of triphenylphosphine, there was produced a polystyrene/triphenyl-phosphonium chloride catalyst.

Example 5

In a procedure similar to Example 1, except that 10 ml of N,N-dimethylbutylamine was replaced by 10 ml of tripropylamine, there was produced a polystyrene/tripropylammonium chloride catalyst.

Example 6

In a procedure similar to Example 1, except that 10 ml of N,N-dimethylbutylamine was replaced by 10 ml of N,N-diethyl-*n*-butylamine, there was produced a polystyrene/diethyl-*n*-butylammonium chloride catalyst.

Example 7

The catalysts of Examples 1 and 2 were compared with similar homogeneous phase transfer catalyst in their ability to cyanate 3-thenylbromide. Into a suitable reactor were placed 150 mmoles of 3-thenylbromide in 200 ml of carbon tetrachloride, 14.7 g (300 mmoles) of sodium cyanide in 50 ml of water was added and the particular phase transfer catalyst shown in Table 1 below was added. All reactions were run at 50°C. except the polymer bound phase transfer catalyst was duplicated at 75°C. in Runs 5 and 6. Samples were taken and analyzed on vapor phase chromatograph at 0.5, 1 and 2 hours for a comparison of the amounts of the percent of 3-thienylacetonitrile present as shown by VPC (see Table 1 below). Run 1 contained tetra-*n*-butylammonium bromide. Run 2 was methyl-tri-*n*-butylammonium chloride, Runs 3 and 5 used the catalyst prepared in Example 1 and Runs 4 and 6 used the catalyst prepared in Example 2. Runs 7 and 8 employed the catalyst prepared in Example 6.

TABLE 1

*Conversion of 3-Thenylbromide to 3-Thienylacetonitrile*

| Run | Catalyst | Amount | Temp., °C. | Time, hr. | % Yield |
|---|---|---|---|---|---|
| 1 | Tetrabutylammonium bromide (comparative homogeneous phase transfer catalyst) | 0.966 g (3 mmoles) | 50 | 0.5 | 58.8 |
| | | | | 1.0 | 76.8 |
| | | | | 2.0 | 85.5 |
| 2 | Methyl-tri-*n*-butyl-ammonium chloride (comparative homogeneous phase transfer catalyst) | 0.706 g (3 mmoles) | 50 | 0.5 | 81.3 |
| | | | | 1.0 | 87.1 |
| | | | | 2.0 | 91.4 |
| 3 | Catalyst from Ex. 1 | 1.0 g | 50 | 0.5 | 1.8 |
| | | | | 1.0 | 22.0 |
| | | | | 2.0 | 41.4 |
| 4 | Catalyst from Ex. 2 | 1.0 g | 50 | 0.5 | 6.0 |
| | | | | 1.0 | 18.9 |
| | | | | 2.0 | 50.7 |
| 5 | Catalyst from Ex. 1 | 1.0 g | 75 | 1.0 | 82.7 |
| | | | | 2.0 | 83.7 |
| 6 | Catalyst from Ex. 2 | 1.0 g | 75 | 1.0 | 80.3 |
| | | | | 2.0 | 92.7 |
| 7 | Catalyst from Ex. 6 | 1.0 g | 50 | 0.5 | 1.3 |
| | | | | 1.0 | 12.0 |
| | | | | 2.0 | 31.2 |
| 8 | Catalyst from Ex. 6 | 1.0 g | 75 | 1.0 | 38.6 |
| | | | | 2.0 | 80.7 |

The reaction of Run 1 turned dark red-brown immediately upon combination of all reactants. An emulsion appeared to be forming also immediately upon combination of reactants. Following the reaction, the solution was extremely difficult to filter, even through a filter aid such as Celite 545 was used after two hours. At the end of the reaction, the reaction mixture was so dark that the phase interface could not be distinguished. The same comments apply to Run 2 having immediate red-brown color formation upon combination, formation of emulsion, extremely difficult filtration and indistinguishable interface. In Run 3, a pale yellow color developed after two hours. No emulsion formed. The solution

filtered rapidly without the use of a filter aid and the aqueous and organic phases could be easily distinguished. In Run 4, the same comments apply as for Run 3 but the color was fainter. The higher temperature runs, Runs 5 and 6, turned dark brown at the end of two hours' reaction. However, no emulsion was formed and the mixtures filtered rapidly without the use of a filter aid. In addition, the phases could be easily distinguished. Thus, it is clear that the higher temperature runs caused little problem with handling the reaction mass and achieved yields as good as or better than the homogeneous catalyst.

Example 8

Recycle of catalysts from Runs 5 and 6 of Example 7.

Although the catalysts produced good reaction yields and were easily separated from the reaction mixture in Example 7, it was desired to determine whether or not the catalysts could be recycled for the obvious economic reasons. Therefore, the catalysts from Runs 5 and 6 of Example 7 were filtered from the reaction mixture as indicated and an additional 1.77 g (0.01 moles) of 3-thenylbromide, 30 ml of carbon tetrachloride, 0.98 g of sodium cyanide, 4 ml of water and 1 g of each catalyst were placed in a reaction vessel. The temperature in each reaction vessel was increased to 72° and samples were taken over a 2-hour period. At the end of one hour, the catalyst from Run 5 of Example 7 had 86.5% yield and the catalyst from Run 6 has 85.3% yield. After 2 hours, the catalyst from Run 5 had 98.2% yield and catalyst from Run 6 had 96.7% yield of 3-thienylacetonitrile, respectively. Thus, both catalysts in accord with Examples 1 and 2 can be recycled and reused without a decrease in yield.

Example 9

The polymer bound phase transfer catalyst of Examples 3, 4, 5 and 6 were compared with the catalyst of Example 1. In their ability to synthesize 1-cyanooctane from 1-bromooctane, each catalyst was placed in a 25 ml round bottom flask with 1.93 g (0.01 moles) of 1-bromooctane, 3 ml of chloroform, 0.49 g (0.01 mole) of sodium cyanide, 1 ml of water and 0.3 g of the particular catalyst. The catalysts were heated at 80°C. in an oil bath for brief periods and samples were taken and analyzed by vapor phase chromatography. The results of these runs are given in Table 2 below:

TABLE 2

*Conversion of 1-Bromooctane to 1-Cyanooctane*

| Catalyst | Time (min.) | Yield of 1-Cyanooctane, % |
|---|---|---|
| From Example 1 | 15 | 22.91 |
| | 45 | 53.6 |
| From Example 3 | 15 | 8.93 |
| | 45 | 18.3 |
| From Example 4 | 15 | 0.71 |
| | 45 | 0.97 |
| From Example 5 | 15 | 19.31 |
| | 45 | 42.1 |
| From Example 6 | 15 | 6.2 |
| | 45 | 24.5 |

Thus, each of the catalysts appear to promote a synthesis of 1-cyanooctane. However, while the catalysts of Example 3 and 4 appear to be less effective than the catalyst of Example 1 over the short time period, catalyst of Example 5 appears to be almost as effective as the catalyst of Example 1.

Example 10

This example will show the amount of catalyst needed, compared to the amount of a homogeneous phase transfer catalyst. In this example, three separate runs were made using the catalyst of Example 2, in which 1.77 g (0.01 mole) of 3-thenylbromide, 8 ml of carbon tetrachloride, 0.98 g (0.02 mole) of sodium cyanide, 2 ml of water and from 10 to 3.3 weight percent of the catalyst of Example 2 were mixed together in a suitable reactor. The mixture was heated at 75°C. for 2 hours

and the yield of 3-thienylacetonitrile was determined by vapor phase chromatography. The result of these runs are shown in Table 3 can be compared with the 90% yield of 3-thenylbromide to 3-thienylacetonitrile after one hour at 55° C. with 7% methyl tri-*n*-butylammonium chloride.

TABLE 3

*Conversion of 3-Thenylbromide to 3-Thienylacetonitrile*
*Variable Study of Amount of Catalyst From Example 2*

| Catalyst From Example 2 | | | | Comparative Catalyst, Methyl Tri-n-butylammonium Chloride |
|---|---|---|---|---|
| Weight Percent | 10 | 7 | 3.3 | 7 |
| Temperature, °C | 75 | 75 | 75 | 55 |
| Percent Yield of 3-Thienylacetonitrile | | | | |
| After 1 hour | 30.2 | 29.7 | 5.3 | — |
| After 2 hours | 57.6 | 57.5 | 21.3 | ~90 |

Note that the 10% and 7% catalyst concentrations appear to be about equal in ability to convert 3-thenylbromide to 3-thienylacetonitrile. The 7% concentration appears to take, however, twice as long to make the conversion than does the equivalent amount of homogeneous phase transfer catalyst.

**Claims**

1. A triphase catalyst for converting bromides into nitriles consisting of a compound of the formula

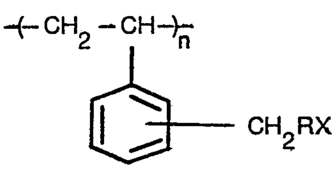

in which R is selected from diethyl-*n*-butylamino, tripropylamino, tributylamino, trialkylphosphino and triarylphosphino; X is chlorine or bromine; and *n* is from 600 to 2,000,000, the term alkyl meaning lower alkyl having from 1 to 4 carbon atoms and the term aryl meaning mononuclear aromatic groups having 6 carbon atoms when unsubstituted and up to 10 carbon atoms when alkyl substituted, and such compounds cross-linked with 1—10% divinylbenzene.

2. A catalyst according to claim 1 wherein R is tripropylamino and X is chlorine.

3. A catalyst according to claim 1 wherein R is tributylamino and X is chlorine.

4. A catalyst according to claim 1 wherein R is tributylphosphino and X is chlorine.

5. A catalyst according to claim 1 wherein R is triphenylphosphino and X is chlorine.

6. A process for preparing a triphase catalyst according to claim 1, characterized by establishing a mixture of chloromethylated styrene polymer in a solvent capable of swelling said styrene polymer, maintaining said mixture for a period of 10 to 30 hours to swell the polymer, reacting the swelled polymer with an amine of phosphine in a suitable solvent under reflux with the resin under a nitrogen atmosphere for 12—36 hours and recovering the resultant triphasic catalyst from the reaction mixture.

7. The process of claim 6 in which said solvent is tetrahydrofuran.

8. The process of claim 6 in which said swelled polymer is reacted with an amine selected from tributylamine and tripropylamine.

9 The process of claim 6 in which said swelled polymer is reacted with a phosphine selected from tributylphosphine and triphenylphosphine.

10. The process of claim 6 in which said chloromethylstyrene polymer is employed in the form of beads which are swelled in tetrahydrofuran for 10 to 30 hours, reacted with tri-n-butylamine at reflux for 24 hours to produce a triphasic catalyst and then recovering the triphasic catalyst produced.

11. Process for converting 3-thenylbromide to 3-thienyl-p-acetonitrile by reacting 3-thenylbromide in an organic solvent with aqueous alkali metal cyanide, characterized by conducting said process in the presence of a catalyst according to claim 1.

12. The process of claim 11 in which R is tripropylamino and X is chlorine.

13. The process of claim 11 in which R is tributylamino and X is chlorine.
14. The process of claim 11 in which R is tributylphosphino and X is chlorine.
15. The process of claim 11 in which R is triphenylphosphino and X is chlorine.

## Revendications

1. Catalyseur triple phase pour la transformation de bromures en nitriles, consistant en un composé de formule

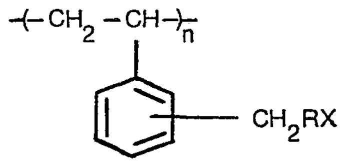

dans laquelle R est choisi entre les groupes diéthyl-*n*-butylamino, tripropylamino, tributylamino, trialkylphosphino et triarylphosphino; X est le chlore ou le brome; et *n* a une valeur de 600 à 2 000 000, le terme alkyle désignant un groupe alkyle inférieur ayant 1 à 4 atomes de carbone et le terme aryle désignant des groupes aromatiques mononucléaires ayant 6 atomes de carbone lorsqu'ils ne sont pas substitués et jusqu'à 10 atomes de carbone lorsqu'ils ont des substituants alkyle, et de tels composés réticulés avec 1—10% de divinylbenzène.

2. Catalyseur suivant la revendication 1, dans lequel R est le groupe tripropylamino et X est le chlore.

3. Catalyseur suivant la revendication 1, dans lequel R est le groupe tributylamino et X est le chlore.

4. Catalyseur suivant la revendication 1, dans lequel R est le groupe tributylphosphino et X est le chlore.

5. Catalyseur suivant la revendication 1, dans lequel R est le groupe triphénylphosphino et X est le chlore.

6. Procédé de préparation d'un catalyseur triple phase suivant la revendication 1, caractérisé en ce qu'il consiste à établir un mélange d'un polymère de styrène chlorométhylé dans un solvant capable de faire gonfler ledit polymère de styrène, à maintenir ledit mélange pendant une période de 10 à 30 heures pour faire gonfler le polymère, à faire réagir le polymère gonflé avec une amine ou une phosphine dans un solvant convenable au reflux par réaction avec la résine sous une atmosphère d'azote pendant 12 à 36 heures et à séparer du mélange réactionnel le catalyseur triple phase résultant.

7. Procédé suivant la revendication 6, dans lequel ledit solvant est le tétrahydrofuranne.

8. Procédé suivant la revendication 6, dans lequel ledit polymère gonflé est amené à réagir avec une amine choisie entre la tributylamine et la tripropylamine.

9. Procédé suivant la revendication 6, dans lequel ledit polymère gonflé est améné à réagir avec une phosphine choisie entre la tributylphosphine et la triphénylphosphine.

10. Procédé suivant la revendication 6, dans lequel ledit polymère de chlorométhylstyrène est utilisé sous la forme de perles qui sont gonflées dans du tétrahydrofuranne pendant 10 à 30 heures, amenées à réagir avec de la tri-n-butylamine au reflux pendant 24 heures pour produire un catalyseur triple phase qui est ensuite recueilli.

11. Procédé pour convertir du bromure de 3-thiényle en 3-thiénylacétonitrile par réaction de bromure de 3-thényle dans un solvant organique avec un cyanure de métal alcalin aqueux, caractérisé en ce qu'il est misen oeuvre en présence d'un catalyseur suivant la revendication 1.

12. Procédé suivant la revendication 11, dans lequel R est le groupe tripropylamino et X est le chlore.

13. Procédé suivant la revendication 11, dans lequel R est le groupe tributylamino et X est le chlore.

14. Procédé suivant la revendication 11, dans lequel R est le groupe tributylphosphino et X est le chlore.

15. Procédé suivant la revendication 11, dans lequel R est le groupe triphénylphosphino et X est le chlore.

## Patentansprüche

1. Drei-Phasen-Katalysator zur Umwandlung von Bromiden in Nitrile, bestehend aus einer Verbindung der Formel:

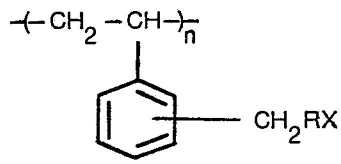

worin R ausgewählt ist aus den Gruppen Diethyl-n-butylamino, Tripropylamino, Tributylamino, Trialkylphosphino und Triarylphosphino, X Chlor oder Brom und n eine Zahl von 600 bis 2.000.000 ist, wobei Alkyl niederes Alkyl mit 1 bis 4 Kohlenstoffatomen bedeutet und Aryl eine monocyclische aromatische Gruppe mit 6 Kohlenstoffatomen, wenn sie nicht substituiert ist, und mit bis zu 10 Kohlenstoffatomen, wenn sie durch Alkyl substituiert ist, als auch solche Verbindungen, die mit 1 bis 10% Divinylbenzol vernetzt sind.

2. Katalysator nach Anspruch 1, worin R Tripropylamino und X Chlor ist.

3. Katalysator nach Anspruch 1, worin R Tributylamino und X Chlor ist.

4. Katalysator nach Anspruch 1, worin R Tributylphosphino und X Chlor ist.

5. Katalysator nach Anspruch 1, worin R Triphenylphosphino und X Chlor ist.

6. Verfahren zur Herstellung eines Drei-Phasen-Katalysators gemäss Anspruch 1, dadurch gekennzeichnet dass eine Mischung aus chlormethyliertem Styrolpolymer in einem Lösungsmittel, welches das Styrolpolymer quellen kann, hergestellt und 10 bis 30 Stunden gehalten wird, um das Polymer zu quellen, das gequollene Polymer mit einem Amin oder Phosphin in einem geeigneten Lösungsmittel unter Rückfluss mit dem Harz in Stickstoffatmosphäre 12 bis 16 Stunden umgesetzt und der so erhaltene Drei-Phasen-Katalysator aus der Reaktionsmischung gewonnen wird.

7. Verfahren nach Anspruch 6, worin das Lösungsmittel Tetrahydrofuran ist.

8. Verfahren nach Anspruch 6, worin das gequollene Polymer mit einem Amin aus der Gruppe Tributylamin und Tripropylamin umgesetzt wird.

9. Verfahren nach Anspruch 6, worin das gequollene Polymer mit einem Phosphin aus der Gruppe Tributylphosphin und Triphenylphosphin umgesetzt wird.

10. Verfahren nach Anspruch 6, worin das Chloromethylstyrol-Polymer in Form von Perlen verwendet wird, welche 10 bis 30 Stunden in Tetrahydrofuran gequollen werden, 24 Stunden bei Rückfluss mit Tri-n-butylamin umgesetzt werden, um einen Drei-Phasen-Katalysator zu erzeugen, und der erzeugte Drei-Phasen-Katalysator gewonnen wird.

11.Verfahren zur Umwandlung von 3-Thenylbromid in 3-Thienylacetonitril durch Umsetzung von 3-Thenylbromid in einem organischen Lösungsmittel mit wässrigem Alkalicyanid, dadurch gekennzeichnet, dass das Verfahren in Gegenwart eines Katalysators gemäss Anspruch 1 durchgeführt wird.

12. Verfahren nach Anspruch 11, worin R Tripropylamino und X Chlor ist.

13. Verfahren nach Anspruch 11, worin R Tributylamino und X Chlor ist.

14. Verfahren nach Anspruch 11, worin R Tributylphosphino und X Chlor ist.

15. Verfahren nach Anspruch 11, worin R Triphenylphosphino und X Chlor ist.